# EUROPEAN PATENT APPLICATION

(11) **EP 4 257 955 A1**
(43) Date of publication of application: **11.10.2023**
(21) Application number: 22166495.6
(22) Date of filing: 04.04.2022
(51) Int. Cl.: G01N 21/51, G01N 21/59, G01N 33/04, G01N 21/53, G01N 33/06, G01N 21/55, G01N 21/17, G01N 15/02

(54) **A METHOD FOR ANALYZING MILK, A METHOD FOR ANALYZING THE CONTENT OF CARBOHYDRATES IN MILK AND A DEVICE FOR ANALYZING MILK**

(71) Applicant: CARAG AG, 6340 Baar (CH)
(72) Inventor: KALYANOV, Alexander, 5507 Mellingen (CH); WOLF, Martin, 8038 Zürich (CH); CHRISTEN, Lukas, 6004 Luzern (CH); ISLER, Helene, 8051 Zürich (CH)
(74) Representative: Grünecker Patent- und Rechtsanwälte PartG mbB

(57) **Abstract**

The present disclosure relates to a method for analyzing milk in which method light is introduced into the milk and signals resulting from a reflectance (R) measurement, from a transmittance (T) measurement and from an unscattered transmittance (UT) measurement are obtained, which signals in combination are electronically processed to output information indicative of at least one parameter of the milk. In other aspects, the present disclosure relates to a method for analyzing the content of carbohydrates in milk by introducing light into the milk and analyzing the light signals coming out from the milk, wherein the wavelength of the light introduced into the milk is not more than 2500 nm, and a device for analyzing milk, which has a first measuring length (4, 14) for measuring transmittance (T) of light through the milk and reflectance (R) of light by the milk, and a second measuring length (24) for measuring unscattered transmittance (UT) of light through the milk, wherein the second measuring length (24) is thinner than the first measuring length (4, 14) by a factor of at least 10.

## Description

The present invention relates to a method for analyzing milk, a method for analyzing the content of carbohydrates in milk and a device for analyzing milk.

Milk in general and breast milk expressed from a mother's breast with a breast pump in particular is analyzed in order to gain information about the constitution of the milk. Generally, breast milk constitutes of approximately 87 % to 88% water, 4 % fat, 6% lactose, 2% oligosaccharides and 1 % protein and vitamins, minerals and other trace elements.

EP 2 793 686 B1 discloses a method for analyzing expressed milk, which determines the fat content in said milk and provides an indication as to the amount of milk remaining in a breast during lactation. In this method, an optical sensor is used to illuminate a sample of milk and to detect the amount of light that is absorbed or scattered by fat globules in the expressed milk. Then, the measured transmittance is compared with data representing the transmittance corresponding to a sample of milk having a known fat content to determine the fat content of said expressed milk. It is known that as a breast empties, the fat content of the breast milk rises. Therefore, the determined fat content is indicative as to the amount of milk remaining in the breast.

The method known from EP 2 793 686 B1 focusses on the fat globules that are the largest particles suspended in the milk. Other constituents are not considered in the analyzing method. This leaves room for improvement.

An object of the present invention is to provide an improved method for analyzing milk. In particular, the present invention wants to provide a method that takes into account the scattering of light caused by at least one other constituent than fat. In this way, the present invention attempts to achieve a higher accuracy and/or precession in determining at least one parameter of the milk. Parameters of the milk can be, for example, the fat content in the milk or the content of at least one other constituent of the milk.

As a solution for this object, the present invention proposes a method according to claim 1. In this method, light is introduced into the milk and signals resulting from a reflectance (R) measurement, from a transmittance (T) measurement and from an unscattered transmittance (UT) measurement are obtained, which signals in combination are processed to output information indicative of at least one parameter of the milk. The transmittance (T) measurement corresponds to a measurement of measuring light emitted from a light source located at one side of a milk sample and detected by a detector located at the opposite side of the milk sample. The reflectance (R) measurement corresponds to a measurement of measuring light emitted from a light source located at one side of a milk sample and detected by a detector located at the same side of the milk sample. Usually, the same milk sample and the same light source is used for the T measurement and the R measurement. The unscattered transmittance (UT) measurement corresponds to a measurement of measuring light emitted from a light source located at one side of a thin milk sample and detected by a detector located at the opposite side of the thin milk sample. The thin milk sample for the UT measurement is thin compared to the milk sample of the T and the R measurement, preferably thinner by a factor of at least 10. Naturally, the term "thin" used before or the term "thickness" used later on refer to the dimension of the milk sample in the direction of light emittance.

Preferably, the thin milk sample for the UT measurement is thinner than the milk sample for the T and the R measurement by a factor that lies in a range of 20 to 400. For example, the thin milk sample for the UT measurement can be thinner than the milk sample for the T and the R measurement by a factor of 40, 100 or 200. The thickness of the milk sample for the T and the R measurement can be appropriately set depending on the particular light source used. Typically, the thickness of the milk sample for the T and the R measurement is not less than 0.5 mm, and preferably not more than 20 mm.

Preferably, the light introduced into the milk is generated by at least one LED and/or at least one laser diode, and more preferably by a plurality of LEDs and/or a plurality of laser diodes generating light of different wavelengths. For the constituents of milk that are smaller than the fat globules, i.e. for casein protein and carbohydrates, the absorption and scattering characteristic can vary depending on the particular wavelength of the light introduced into the milk. Therefore, using a plurality of LEDs and/or laser diodes generating light of different wavelengths can improve the analysis. Alternatively, a light source having a wide emission range can be used in combination with a detector comprising a spectrometer and/or at least one bandpass filter.

Preferably, the light introduced into the milk has a wavelength of not more than 2500 nm. Further, the light introduced into the milk preferably has a wavelength of not less than 400 nm. More preferably, the light introduced into the milk has a wavelength of not more than 1200 nm. LEDs generating light having a wavelength in this range are comparably inexpensive and are therefore preferably used. Most preferably, approximately ten different LEDs and/or laser diodes are used.

The signal resulting from the UT measurement is preferably extrapolated to obtain a UT result corresponding to a milk sample having the same thickness as the milk sample of the T measurement and the R measurement. Said UT result can be computed with the transmittance and reflectance for calculating the ratio of scattered light. Preferably, a plurality of UT measurements are performed with different sample thicknesses within the above-mentioned relative range. This can improve extrapolation.

Preferably, the milk is analyzed as un-homogenized milk, which has the advantage that the milk does not need to be homogenized and the number of handling steps that need to be carried out for the method according to the present invention is reduced. Further, the milk is exposed to less treatment if homogenization is omitted, which can have beneficial effects on the health of the consumer of the milk.

Preferably, the signals resulting from the reflectance (R) measurement, from the transmittance (T) measurement and from the unscattered transmittance (UT) measurement are processed using an inverse adding doubling (IAD) algorithm. The IAD algorithm employs the adding-doubling method to compute the optical properties of slabs of material from reflectance, transmittance and unscattered transmittance measurements. It repeatedly guesses the optical properties and compares the expected observable measurement values to the actual measurement values (T, R, UT) until a match is found. Optical properties are output after processing the signals via the IAD algorithm. Those optical properties can be an absorption coefficient (µₐ) and/or a reduced scattering coefficient (µ'ₛ) and/or an anisotropy factor (g).

Preferably, scattering outputs resulting from the IAD computation are processed using Mie scattering formulas. Mie scattering formulas are most useful in situations where the size of the scattering particles is comparable to the wavelength of the light, rather than much smaller or much larger. As mentioned above, wavelengths of not more than 2500 nm are preferably used. Therefore, the wavelength of the light introduced into the milk is comparable to the size of the fat and casein particles in the milk and Mie scattering formulas can be used to describe the scattering behaviour of light due to fat and casein micelles in the milk. More preferably, the optical properties obtained using IAD are inputted into the Mie scattering formulas in order to determine a particle size distribution for the analyzed milk. Further, a mathematical model can be used which receives the optical properties obtained using IAD and the particle size distribution obtained using Mie scattering formulas as input and outputs at least one parameter relating to the milk composition. The parameters relating to the milk composition typically are fat content, protein content, carbohydrate content, particle size and particle size distribution. The particle size distribution obtained using Mie scattering formulas allows to assign an amount of scattering to the carbohydrate dissolved in the water constituent of the milk.

Thus, the method for analyzing milk preferably analyzes the content of fat, proteins and/or carbohydrates in the milk and/or the particle size and/or the particle size distribution of fat, and/or proteins in the milk. As it is principally known from EP 2 793 686 B1 for the fat content, the measured and/or processed signals can be compared to data of a lookup table in order to determine the desired parameters.

In another aspect, the present invention relates to a method for analyzing the content of carbohydrates in milk by introducing light into the milk and analyzing the light signals coming out from the milk, wherein the wavelength of the light introduced into the milk is not more than 2500 nm, and preferably not more than 2000 nm. Further, the signals coming out from the milk are preferably generated by the above-mentioned T, R and UT measurements and preferably analyzed according to the above-mentioned method for analyzing milk.

In another aspect, the present invention relates to a device for analyzing milk, which has a first measuring length for measuring transmittance and/or reflectance and a second measuring length for measuring unscattered transmittance, wherein the second measuring length is thinner than the first measuring length by a factor of at least 10. Preferably, said factor lies in a range of 20 to 400. More preferably, the device has a second measuring length and at least one more measuring length for measuring unscattered transmittance, wherein the second measuring length and the at least one more measuring length are thinner than the first measuring length by a different factor within the above-mention preferred range. Further, the device preferably has a processing unit for processing the signals generated by the T, the R and the UT measurements and for outputting at least one parameter of the milk. In particular, the device can be configured to carry out the above-mentioned methods for analyzing milk. The device can contain a funnel-like channel where a milk layer gets thinner as the channel tapers, or the device can comprise a measurement cell and a mechanical unit for adjusting the shape of the measurement cell in order to change the thickness of a milk layer in the measurement cell.

The device can be a stand alone device, or be integrated into or connected to a bottle, a baby bottle or an apparatus for expressing milk from a breast, or a feeding device, or a milk fortification device. For example, the information gained by the method about the content of the constituents of the milk according to the present invention can be used for milk fortification in order to adjust the milk composition individually to the nutritional requirement of a particular infant. Some breastfeeding mothers like to keep track of the nutritional information relating to their breast milk. Thus, as another example, the information gained by the method about the content of the constituents of the milk according to the present invention could be stored over a specific period of time and displayed, preferably on a display of a smart phone or the like. Breastfeeding mothers may even use this information to adjust their diet.

Further details and advantages of the present invention will be obtained from the following description of an embodiment and the accompanying drawings, in which:
- Figure 1a: illustrates the transmittance (T) measurement,
- Figure 1b: illustrates the reflectance (R) measurement,
- Figure 1c: illustrates a combined transmittance (T) and reflectance (R) measurement,
- Figure 1d: illustrates the unscattered transmittance (UT) measurement,
- Figure 2: illustrates the processing of the measured signals, and
- Figure 3: illustrates an add-on of the processing shown in Figure 2.

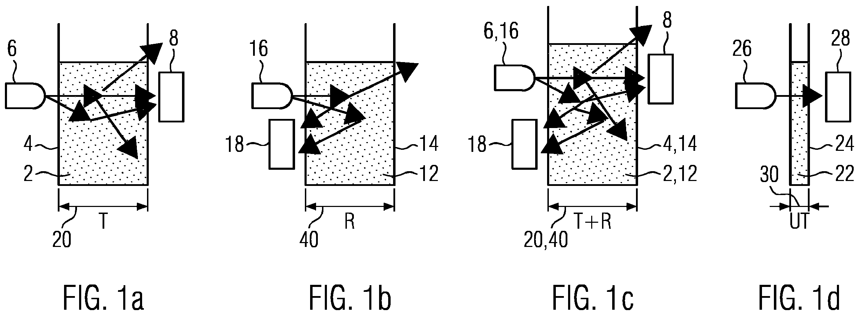

Figure 1a illustrates a milk sample 2 in a measuring length 4, an LED 6 at one side of the milk sample 2 for illuminating the milk sample 2 and a first light detector 8 at the opposite side of the milk sample 2 for detecting light that is transmitted through the milk sample 2.

Figure 1b illustrates a milk sample 12 in a measuring length 14, an LED 16 at one side of the milk sample 12 for illuminating the milk sample 12 and a second light detector 18 at the same side of the milk sample 12 for detecting light that is reflected by the milk sample 12. Naturally, the LED 6 and the LED 16 may be the same LED, the milk sample 2 and the milk sample 12 may be the same milk sample and the measuring length 4 and the measuring length 14 may be the same measuring length as illustrated in Figure 1c. The measuring length 4 and the measuring length 14 have a thickness referenced by reference numerals 20, 40. Preferably, the thicknesses 20 and 40 are equal as illustrated in Figure 1c. However, they can also be different, and the difference can be compensated by mathematical extrapolation.

Figure 1d illustrates a thin milk sample 22 in a second measuring length 24, an LED 26 at one side of the thin milk sample 22 for illuminating the milk sample 22 and a third light detector 28 at the opposite side of the thin milk sample 22 for detecting light that is transmitted through the thin milk sample 22. The second measuring length 24 has a thickness referenced by reference numeral 30 that is thinner than the thickness 20, 40 of the measuring lengths 4, 14 by a factor of twenty to four hundred. The LEDs 6, 16, 26 generate light of the same wavelength.

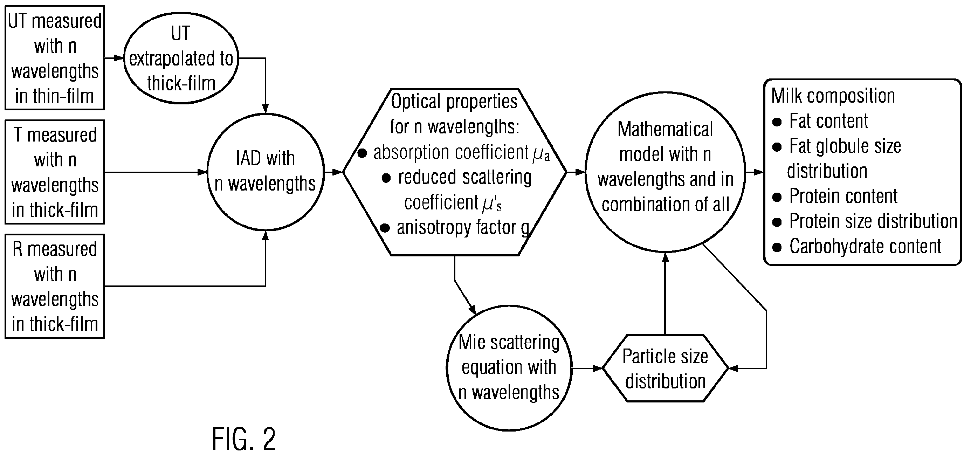

Figure 2 illustrates the processing of the measured signals, wherein each UT, T and R measurement is carried out with n LEDs having different wavelengths, n being a natural number. The UT measurements may be performed, for each wavelength, for a plurality of measuring lengths having different thicknesses, wherein the results of the UT measurements are extrapolated to correspond to a result for a measuring length having the same thickness as the measuring length of the T and the R measurement. The extrapolated UT measurement result and the measured T and R signals are input into the inverse adding doubling (IAD) algorithm and this IAD algorithm outputs optical properties, such as an absorption coefficient, a reduced scattering coefficient and an anisotropy factor, which are inserted into Mie scattering formulas in order to determine a particle size distribution. The particle size distribution determined with the Mie scattering formulas and the optical properties output from the IAD algorithm are then input to a mathematical model to determine the milk composition as a result of the analysis. Parameters indicative of the milk composition are fat content, fat globule size distribution, protein content, protein size distribution, and carbohydrate content.

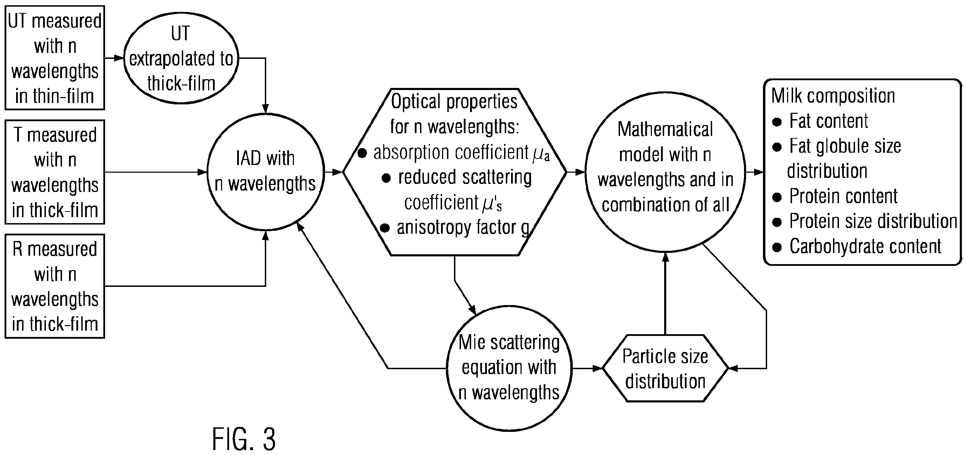

Figure 3 illustrates an add-on of the processing shown in Figure 2, wherein an additional loop between the IAD algorithm and the Mie scattering formulas is added. That is, according to the add-on, the IAD algorithm computes a reduced scattering coefficient µ'ₛ and an absorption coefficient µₐ. Subsequently, a corrected reduced scattering coefficient µ'ₛ is computed using the Mie scattering formulas, and the corrected reduced scattering coefficient µ'ₛ is input into the IAD algorithm to compute again µₐ.

### List of reference signs

- 2, 12: milk sample
- 4, 14, 24: measuring length
- 6, 16, 26: LED
- 8, 18, 28: light detector
- 20: thickness of measuring length 4
- 22: thin milk sample
- 30: thickness of measuring length 24
- 40: thickness of measuring length 14

## Claims

1. A method for analyzing milk in which method light is introduced into the milk and signals resulting from a reflectance (R) measurement, from a transmittance (T) measurement and from an unscattered transmittance (UT) measurement are obtained, which signals in combination are electronically processed to output information indicative of at least one parameter of the milk.

2. The method of claim 1, wherein the light introduced into the milk is generated by at least one LED (6, 16, 26) and/or at least one laser diode and preferably by a plurality of LEDs and/or a plurality of laser diodes generating light of different wavelengths.

3. The method of claim 1, wherein the light introduced into the milk is generated by at least one light source having a wide emission range and wherein the light emanating from the milk is detected by a detector comprising a spectrometer and/or at least one bandpass filter.

4. The method of claim 1, 2, or 3, wherein the light introduced into the milk has a wavelength of not more than 2500 nm and preferably not less than 400 nm.

5. The method of any one of the preceding claims, wherein the milk is analyzed as un-homogenized milk.

6. The method of any one of the preceding claims, wherein the signal indicative of the unscattered transmittance (UT) is obtained by transmitting light through a thin milk sample (22) with a measuring length (24) having a thickness (30) that is thinner than a thickness (20, 40) of a measuring length (4, 14) of a milk sample (2, 12) illuminated in the T measurement and the R measurement by a factor of at least 10, and preferably by a factor that lies in a range of 20 to 400.

7. The method of any one of the preceding claims, wherein the signals resulting from the R measurement, from the T measurement and from the UT measurement are processed using an inverse adding doubling (IAD) algorithm.

8. The method of any one of the preceding claims, wherein optical properties deduced from the signals of the R measurement, the T measurement and the UT measurement are processed using Mie-scattering formulas.

9. The method of any one of the preceding claims, wherein the content of fat, proteins and/or carbohydrates in the milk is analyzed.

10. The method of any one of the preceding claims, wherein the particle size and/or the particle size distribution of fat and/or proteins in the milk is analyzed.

11. A method for analyzing the content of carbohydrates in milk by introducing light into the milk and analyzing the light signals coming out from the milk, wherein the wavelength of the light introduced into the milk is not more than 2500 nm, and preferably not more than 2000 nm.

12. A device for analyzing milk, which has a first measuring length (4, 14) for measuring transmittance (T) of light through the milk and reflectance (R) of light by the milk, and a second measuring length (24) for measuring unscattered transmittance (UT) of light through the milk, wherein the second measuring length (24) is thinner than the first measuring length (4, 14) by a factor of at least 10 and preferably by a factor that lies in a range of 20 to 400.

## Amended claims

### Amended claims in accordance with Rule 137(2) EPC.

1. A method for analyzing milk in which method light is introduced into the milk and signals resulting from a reflectance (R) measurement, from a transmittance (T) measurement and from an unscattered transmittance (UT) measurement are obtained, which signals in combination are electronically processed to output information indicative of at least one parameter of the milk, wherein the signal indicative of the unscattered transmittance (UT) is obtained by transmitting light through a thin milk sample (22) with a measuring length (24) having a thickness (30) that is thinner than a thickness (20, 40) of a measuring length (4, 14) of a milk sample (2, 12) illuminated in the T measurement and the R measurement by a factor of at least 10.

2. The method of claim 1, wherein the light introduced into the milk is generated by at least one LED (6, 16, 26) and/or at least one laser diode and preferably by a plurality of LEDs and/or a plurality of laser diodes generating light of different wavelengths.

3. The method of claim 1, wherein the light introduced into the milk is generated by at least one light source having a wide emission range and wherein the light emanating from the milk is detected by a detector comprising a spectrometer and/or at least one bandpass filter.

4. The method of claim 1, 2, or 3, wherein the light introduced into the milk has a wavelength of not more than 2500 nm and preferably not less than 400 nm.

5. The method of any one of the preceding claims, wherein the milk is analyzed as un-homogenized milk.

6. The method of any one of the preceding claims, wherein the signal indicative of the unscattered transmittance (UT) is obtained by transmitting light through a thin milk sample (22) with a measuring length (24) having a thickness (30) that is thinner than a thickness (20, 40) of a measuring length (4, 14) of a milk sample (2, 12) illuminated in the T measurement and the R measurement by a factor that lies in a range of 20 to 400.

7. The method of any one of the preceding claims, wherein the signals resulting from the R measurement, from the T measurement and from the UT measurement are processed using an inverse adding doubling (IAD) algorithm.

8. The method of any one of the preceding claims, wherein optical properties deduced from the signals of the R measurement, the T measurement and the UT measurement are processed using Mie-scattering formulas.

9. The method of any one of the preceding claims, wherein the content of fat, proteins and/or carbohydrates in the milk is analyzed.

10. The method of any one of the preceding claims, wherein the particle size and/or the particle size distribution of fat and/or proteins in the milk is analyzed.

11. A method for analyzing the content of carbohydrates in milk by introducing light into the milk and analyzing the light signals coming out from the milk, wherein the wavelength of the light introduced into the milk is not more than 2500 nm, and preferably not more than 2000 nm.

12. A device for analyzing milk, which has a first measuring length (4, 14) for measuring transmittance (T) of light through the milk and reflectance (R) of light by the milk, and a second measuring length (24) for measuring unscattered transmittance (UT) of light through the milk, wherein the second measuring length (24) is thinner than the first measuring length (4, 14) by a factor of at least 10 and preferably by a factor that lies in a range of 20 to 400.
